# EUROPÄISCHE PATENTSCHRIFT

(11) **EP 0 760 393 B1**
(45) Veröffentlichungstag und Bekanntmachung des Hinweises auf die Patenterteilung: **04.06.2003**
(21) Anmeldenummer: 96112637.2
(22) Anmeldetag: 05.08.1996
(51) Int. Cl.: C11C 1/00, B01D 15/08, C07C 51/47, C07C 67/56

(54) **Chromatographie-Verfahren**
Chromatography process
Procédé de chromatographie

(30) Priorität: 17.08.1995 CH 235595
(43) Veröffentlichungstag der Anmeldung: 05.03.1997
(73) Patentinhaber: F. HOFFMANN-LA ROCHE AG, 4070 Basel (CH)
(72) Erfinder: Brunner, Gerd, 21075 Hamburg (DE); Reichmann, Frank, 31303 Burgdorf (DE)
(74) Vertreter: Kellenberger, Marcus, Dr.

(56) Entgegenhaltungen:
- EP-A- 0 416 561
- EP-A- 0 558 974
- WO-A-94/25552
- DE-C- 741 359
- US-A- 4 880 543
- DATABASE WPI Section Ch, Week 8805 Derwent Publications Ltd., London, GB; Class B05, AN 88-033007 XP002038198 & JP 62 292 744 A (EISAI CO LTD) , 19.Dezember 1987

## Beschreibung

Die vorliegende Erfindung betrifft ein Verfahren zum Gewinnen von ungesättigten, gegebenenfalls derivatisierten Fettsäuren aus Gemischen von diesen mit anderen Komponenten, z.B. gesättigten, gegebenenfalls derivatisierten Fettsäuren, durch Säulenchromatographie.

Seit langem herrscht ein ernährungs- und medizinwissenschaftliches Interesse an ungesättigten, insbesondere an polyungesättigten, Fettsäuren (engl. polyunsaturated fatty acids, als "PUFAs" abgekürzt). Bekanntlich sind gewisse Fettsäuren, insbesondere die PUFAs, Vorläufer für Prostanoid-Verbindungen, u.a. Prostaglandine, Thromboxane und Leukotriene, die eine wichtige biologische Rolle u.a. bei Thrombozytenaggregation, Entzündung sowie Allergien spielen. Insbesondere sind die beiden "ω-3"-PUFAs (bei denen sich die erste Doppelbindung am dritten Kohlenstoffatom von der endständigen Methylgruppe der Säure befindet) Eicosapentaensäure ("EPA") und Docosahexaensäure ("DHA") von Nutzen als Wirkstoffe bei der Behandlung und Vorbeugung von "Zivilisationskrankheiten", insbesondere der Herz-Kreislauf-Krankheiten, z.B. Herzinfarkt und erhöhter Cholesterinwert. Ebenfalls bekannt ist der Befund, dass die ω-3- und auch noch die ω-6-PUFAs (mit der ersten Doppelbindung am sechsten Kohlenstoffatom) eine essentielle Rolle bei der Retina-, Gehirn- und allgemeinen Entwicklung von Säuglingen spielen. So wurden ω-3- und ω-6-PUFAs in der Muttermilch nachgewiesen; es gilt als gesichert, dass mit Muttermilch gestillte Kinder sich deutlich schneller entwickeln als vergleichbare Kinder, die nicht gesäugt werden. Darüber hinaus werden insbesondere Eicosapentaensäure und Docosahexaensäure auf ihre positiven Wirkungen bei der Bekämpfung der Arteriosklerose, der Senkung des Cholesterin- und Blutfettspiegels sowie der Verhinderung von Thrombozytenaggregationen, chronischen Entzündungen, wie beispielsweise rheumatoide Arthritis und Neurodermatitis, und Allergien gegenwärtig intensiv untersucht.

Natürliche Fette und Oele, insbesondere Fisch-, Seetier- und pflanzliche Oele, sind wichtige Quellen der ω-3- und ω-6-PUFAs, die vor allem in Form ihrer Glyceride und Phospholipide vorkommen und von vielen unerwünschten Nebenprodukten und Verunreinigungen begleitet sind. Die obenerwähnten ω-3-PUFAs Eicosapentaensäure und Docosahexaensäure sind hauptsächlich in Fischölen enthalten, während beispielsweise die ω-6-PUFAs Arachidonsäure und Linolsäure vor allem in tierischen Fetten bzw. pflanzlichen Oelen, z.B. Maisöl, vorkommen. Angesichts der obenerwähnten Vorteile bei der Einnahme bzw. Verabreichung von PUFAs und der Tatsache, dass der Gesamtgehalt an erwünschten langkettigen PUFAs in alimentären Fetten und Oelen im Bereich von etwa 10 bis 21% liegt (z.B. an ω-3- und ω-6-PUFAs in Fischöl im Bereich von etwa 12 bis 18%), müssten für die empfohlene Dosierung der ω-3-PUFAs täglich grosse Mengen Fisch mit der Nahrung aufgenommen werden. Dies ist jedoch aus den unterschiedlichsten Gründen nicht praktikabel. Daher sind einige Produzenten dazu übergegangen, neben dem schon seit Jahrzehnten auf dem Markt erhältlichen Fischölen in natürlicher Konzentration Kapseln zu vertreiben, die angereicherte PUFAs in möglichst reiner Form, als Ester oder als resubstituierte Triglyceride enthalten. Der Wunsch nach möglichst reinen einzelnen PUFAs bleibt bestehen.

Durch alkalische Hydrolyse der die PUFAs enthaltenden Gemische, z.B. der Fisch-, Seetier- oder pflanzlichen Oele selber oder raffinierter Formen davon, und anschliessende Umesterung mit Alkoholen, insbesondere mit Ethanol, werden u.a. die entsprechenden Alkylester erhalten. Bekanntlich kann die darauffolgende technische Trennung des Alkylestergemisches u.a. mittels Gegenstromextraktion mit einem überkritischen Gas, insbesondere Kohlendioxid, erfolgen, wobei eine weitgehende Trennung nach der Anzahl der Kohlenstoffatome möglich ist. Auf diese Weise werden Fraktionen mit relativ hohen Reinheitsgraden an PUFAs der gleichen oder der nicht weit auseinanderliegenden Kohlenstoffanzahl, z.B. ein 60%-reines Gemisch von Eicosapentaensäure (C₂₀) und Docosahexaensäure (C₂₂), erhalten. Eine gezielte Trennung nach dem Sättigungsgrad (Anzahl der Doppelbindungen) ist auf diese Weise allerdings erfahrungsgemäss nicht möglich. Um jedoch physiologische Wirksamkeiten der einzelnen PUFAs untersuchen zu können, sind Fraktionen bedeutend höherer Reinheit erforderlich. Infolge der bisherigen Unzulänglichkeiten der Trennmethoden konnte u.a. noch nicht geklärt werden, ob beispielsweise die einzelnen ω-3-PUFAs Eicosapentaensäure und Docosahexaensäure getrennt oder im Wechselspiel wirken, was auch für die Docosapentaensäure gilt, die stets zwangsläufig mit Docosahexaensäure verabreicht wird.

Mit einer Trennung von PUFAs, insbesondere von den ernährungsund medizinwissenschaftlich interessanten PUFAs, welche mindestens sechzehn Kohlenstoffatome im Molekül aufweisen, sowohl nach der Anzahl der Kohlenstoffatome als auch nach der Anzahl der Doppelbindungen stünden einzelne PUFAs zur Verfügung, die nicht nur eine Ergänzung zur täglichen Nahrung darstellen würden, sondern auch gezielt medikamentös verabreicht werden könnten.

Einige Veröffentlichungen, die grösstenteils auf Arbeiten der Gruppen um M. Perrut basieren [siehe beispielsweise LC-GC (Magazine of Liquid and Gas Chromatography) 6, 10 (1988), 914], liegen auf dem Gebiet der präparativen überkritischen Flüssigchromatographie (engl. supercritical fluid chromatography, als "SFC" abgekürzt) vor. Gemäss dieser Veröffentlichungen können aus Fischöl gewonnene, in die entsprechenden Ethylester umgeesterte Docosapentaensäure und Docosahexaensäure auf gepackten Säulen mittels SFC präparativ getrennt werden. Unter Verwendung einer mit Silika gepackten Säule konnten Reinheiten von Eicosapentaensäure und Docosahexaensäure von 96% bzw. 85% erzielt werden. Hinweise auf die Trennung von Docosapentaensäure werden nicht gegeben, was angesichts der relativ geringen erzielten Reinheit von 85% für die Docosahexaensäure darauf schliessen lässt, dass die Docosapentaensäure nicht auf diese Weise abgetrennt werden konnte.

Aus der europäischen Patentpublikation (EP) 558.974 ist ein SFCsäulenchromatographisches Verfahren zum Gewinnen von ungesättigten Fettsäuren oder von Derivaten davon aus diese enthaltenden pflanzlichen oder tierischen Gemischen bekannt geworden. In diesem Verfahren wird als mobile Phase überkritisches oder flüssiges Kohlendioxid, und als stationäre Phase eine näher definierte belegte Phase verwendet. Bei der stationären Phase handelt es sich um ein in der Regel aus Silikagel oder Aluminiumoxid bestehendes Grundgerüst, das zwingend mit einer Belegungs-Phase versehen ist, welche freie Elektronenpaare und/oder Mehrfachbindungen aufweist, wie diese beispielsweise in einem eine Aminogruppe (z.B. in Aminopropyl) oder eine Nitrogruppe bzw. eine Phenylgruppe oder eine Cyangruppe (z.B. in Cyanpropyl) enthaltenden Stoff vorkommen. Dass eine wirksame Abtrennung der einzelnen PUFAs durch SFC-Säulenchromatographie mit Kohlendioxid als mobiler Phase, jedoch unter Verwendung eines unbelegten Grundgerüstes, insbesondere Aluminiumoxid, erreicht wird, geht aus der EP 558.974 in keiner Weise hervor.

Aufgabe der vorliegenden Erfindung war es, ein neuartiges Verfahren zum Gewinnen von wertvollen ungesättigten Fettsäuren, als solchen oder als Derivaten, insbesondere im voraus hergestellten Niederalkylestern dieser Fettsäuren, aus einem Gemisch der Fettsäuren und/oder Fettsäureester durch Säulenchromatographie zur Verfügung zu stellen, und zwar ein Verfahren, das die Nachteile des Standes der Technik grösstenteils nicht aufweist. Bei dem erfindungsgemässen Verfahren handelt es sich um ein Verfahren zum Gewinnen ungesättigter Fettsäuren mit mindestens sechzehn Kohlenstoffatomen im Molekül oder eines Derivats einer solchen Fettsäure aus einem Gemisch von Fettsäuren und/oder Fettsäurederivaten durch Säulenchromatographie mit überkritischem oder flüssigem Kohlendioxid als mobiler Phase, das dadurch gekennzeichnet ist, dass als stationäre Phase mit Alkali vorbehandeltes Aluminiumoxid verwendet wird.

Im Prinzip wird das erfindungsgemässe Verfahren so durchgeführt, dass man das gewünschtenfalls bereits unter Druck stehende Gemisch von Fettsäuren und/oder Fettsäurederivaten mit der mobilen Phase des überkritischen oder flüssigen Kohlendioxids zusammenbringt, das Ganze, eventuell gefolgt von weiterer mobiler Phase, auf die mit der obenerwähnten stationären Phase gepackte Chromatographiesäule gibt und dann durchströmen (eluieren) lässt, wobei das Eluieren unter den gewählten Temperatur- und Druckbedingungen erfolgt und aufgrund der starken Wechselwirkungen zwischen der stationären Phase und den einzelnen Bestandteilen des Gemisches eine zeitliche Auftrennung dieser Bestandteile erreicht wird, die aus der Säule nacheinander eluierenden, in Kohlendioxid gelösten Bestandteile (Eluate) nach sequentieller Detektion (Bestimmung) in durch das Detektionsmittel bestimmten Auffanggefässen sammelt und das Kohlendioxid vom jeweiligen Sammelgut durch Dekomprimieren (Verflüchtigung) entfernt, so dass sich schliesslich die erhaltenen, abgetrennten Bestandteile oder "Fraktionen"-(u.a. die erwünschten ungesättigten Fettsäuren bzw. Fettsäurederivate) kohlendioxidfrei in den einzelnen Auffanggefässen befinden. Gewünschtenfalls kann nach dem Eluieren und Austritt aus der Säule das Eluat einem oder mehreren weiteren derartigen chromatographischen Vorgängen unterworfen werden, um eine noch grössere Aufteilung der Bestandteile zu erzielen. Dies gilt auch für eine beliebige Fraktion, falls deren Reinheit der angestrebten nicht entspricht.

Wird nach Durchführung des erfindungsgemässen Verfahrens ein ursprünglich im Gemisch vorhandenes Derivat, z.B. ein Niederalkylester oder ein natürlich vorkommendes Triglycerid, einer ungesättigten Fettsäure mit mindestens sechzehn Kohlenstoffatomen aus dem verwendeten Gemisch gewonnen, jedoch die Fettsäure selber erwünscht wird, so kann diese auf konventionelle Weise aus dem Derivat erhalten werden, z.B. durch Verseifung des Niederalkylesters bzw. Triglycerids. Auch nach einer solchen chemischen Behandlung kann im Falle des Erhalts einer ungenügend angereicherten ungesättigten Fettsäure das diesbezügliche Produkt dem erfindungsgemässen Verfahrens unterworfen werden, bis beim resultierenden Eluierungsprodukt die angestrebte Reinheit erreicht worden ist.

Als Gemisch von Fettsäuren und/oder Fettsäurenderivaten eignet sich jedes beliebige Gemisch, das zumindest eine ungesättigte Fettsäure mit mindestens sechzehn Kohlenstoffatomen im Molekül oder ein Derivat davon enthält. So kann man beispielsweise Oele oder Fette pflanzlicher oder tierischer (einschliesslich seetierischer) Herkunft verwenden, die u.a. die erwünschten Fettsäuren in Form von Triglyceriden, Amiden, Phospholipiden, Laktonen und Salzen, und sonst andere Fettsäuren und Derivate, wie Triglyceride usw., davon, Sterole, z.B. Cholesterol, Vitamine, z.B. Tocopherole, und Materialien, die normalerweise als Verunreinigungen angesehen sind, z.B. Polychlorbiphenyl (PCB), polyaromatische Kohlenwasserstoffe (PAH), Pestizide, Dioxine, Schwermetalle, Oxidations- und Zersetzungsprodukte von gesättigten Fettsäuren oder deren Derivaten, aus bisher verwendeten Konzentrations- oder Reinigungsstufen zurückgebliebene Lösungsmittel oder Reagenzien usw. enthalten. Im erfindungsgemäßen verfahren setzt man ein im voraus ggf. raffiniertes und chemisch behandeltes, verestertes oder umgeestertes Rohmaterial (Oel oder Fett) ein. Das entsprechende, gegebenenfalls im voraus raffinierte Oel oder Fett wird einer sauren oder basischen Hydrolyse unterworfen, wobei die darin enthaltenen Triglyceride in die entsprechenden Säuren übergeführt werden, und mit einem C₁₋₆-Alkylalkohol, bevorzugt mit Ethanol, verestert, wobei die Triglyceride der ungesättigten und gesättigten Fettsäuren in die entsprechenden Niederalkylester umgeestert werden. Die Hydrolyse bzw. Veresterung kann auf konventionelle Weise erfolgen. Bevorzugte Rohmaterialien, die gegebenenfalls im voraus raffiniert und/oder chemisch behandelt worden sind, sind Fischöle, z.B. Sardinen- und Thunfischöl, da diese wertvolle Quellen der höchst begehrten Eicosapentaensäure und Docosahexaensäure sind, sowie tierische Fette und pflanzliche Oele, z.B. Maisöl, da diese ihrerseits wertvolle Quellen der ebenfalls begehrten Arachidonsäure bzw. Linolsäure sind, insbesondere die Fischöle.

Das Gemisch von Fettsäuren und/oder Fettsäure estern wird normalerweise ohne Verdünnung zusammen mit dem überkritischen oder flüssigen Kohlendioxid auf die mit der erfindungsgemäss verwendeten stationären Phase gepackte Chromatographiesäule gegeben, kann allerdings im voraus in einem geeigneten Lösungsmittel, z.B. einem niederen Alkan, vorzugsweise n-Hexan, gelöst werden. Vorzugsweise wird jedoch das Gemisch unverdünnt eingesetzt.

Bei dem im erfindungsgemässen Verfahren verwendeten überkritischen Kohlendioxid handelt es sich bekanntlich um die Form von Kohlendioxid, die bei einer Temperatur von mindestens etwa 31°C und bei einem Druck von mindestens etwa 73 bar gehalten wird und weder rein flüssig noch rein gasförmig, sondern ein Hybrid der beiden physikalischen Formen ist. Das im erfindungsgemässen Verfahren als Alternative verwendbare flüssige Kohlendioxid weist eine Temperatur von weniger als etwa 31°C und einen Druck, der oberhalb von etwa 73 bar liegt, auf. Die Vorteile der Verwendung von Kohlendioxid bestehen in dessen Nichttoxizität, Nichtflammbarkeit und leichter Entfernung durch Dekomprimieren der aufgefangenen Eluate, ohne dass ein potentiell schädigender Rückstand mit der abgetrennten ungesättigten Fettsäure bzw. mit einem Derivat davon bleibt. Ferner ist das Kohlendioxid in hoher Reinheit und kostengünstig breit erhältlich und lässt sich gewünschtenfalls mit einem organischen Cosolvens ("Modifier"), z.B. dem bereits oben erwähnten n-Hexan, aber auch noch einem niederen Alkanol, etwa Methanol oder Ethanol, und einem niederen aliphatischen Keton, etwa Aceton, als Teil der mobilen Phase verwenden. Da die kritische Temperatur von Kohlendioxid nicht viel höher ist als die Raumtemperatur und die erfindungsgemäss zu gewinnenden ungesättigten Fettsäuren bzw. Fettsäurederivate temperaturempfindlich (thermolabil) sind, eignet sich Kohlendioxid auch aus diesen Gründen hervorragend als im erfindungsgemässen Verfahren verwendete mobile Phase.

Das im erfindungsgemässen Verfahren als stationäre Phase - und für die Erfindung kennzeichnend - verwendete Aluminiumoxid liegt zweckmässigerweise als möglichst homogen gepackte, ungleichmässige oder, vorzugsweise, kugelförmige (sphärische) Partikel vor, deren Partikelgrösse etwa 5 bis 25 µm beträgt. Solches kugelförmige Aluminiumoxid ist im Handel leicht erhältlich. Als Beispiele kommerziell erhältlicher Aluminiumoxide seien Aluspher®Al und Spherisorb Alumina erwähnt; das erstere weist eine spezifische Oberfläche S_{BET} von 170 m²/g, ein Porenvolumen Vp von 0,5 ml/g, einen Porendurchmesser D von 100 Å und eine Partikelgrösse dp₅₀ von 5 µm auf, während das letztere eine S_{BET} von 93 m²/g, einen D von 130 Å und eine dp₅₀ von ebenfalls 5 µm aufweist. Gebrochene Materialien werden von verschiedenen Herstellern angeboten, z.B. ICN Biomedicabs, Inc. Diese Aluminiumoxide weisen Korngrössenverteilungen von 3-6 µm, 7-12 µm, 10-18 µm oder 18-32 µm auf. Die spezifische Oberfläche S_{BET} dieser Materialien beträgt 200 m²/g; die Porenvolumen und Porendurchmesser liegen im Bereich sphärischer Materialien.

Das Aluminiumoxid wird vor Verwendung einer alkalischen Vorbehandlung unterworfen. Bei dieser Vorbehandlung handelt es sich im Prinzip um den Kontakt des partikelförmigen Aluminiumoxids mit einer wässrigen Lösung eines Alkalimetall- oder Erdalkalimetallhydroxids, wie beispielsweise Natrium-, Kalium- bzw. Calciumhydroxid, im pH-Bereich von etwa 10 bis etwa 13, über mehrere Stunden, zweckmässigerweise über etwa 8 bis etwa 20 Stunden. Die Konzentration der wässrigen Alkalimetall- oder Erdalkalimetallhydroxidlösung liegt zweckmässigerweise im Bereich von etwa 0,01 M bis etwa 0,1 M. Gewünschtenfalls kann die wässrige Alkalimetall- oder Erdalkalimetallhydroxidlösung mit einem wassermischbaren oder wasserlöslichen organischen Lösungsmittel ergänzt werden, um die Viskosität der Lösung auf einen arbeitstechnisch praktikablen Wert einzustellen. Dies wird beispielsweise mit dem polaren organischen Lösungsmittel Acetonitril oder Aceton erreicht. Als besonders geeignet hat sich das 10:90 (V/V)-Gemisch von Acetonitril und 0,1 M wässriger Natriumhydroxidlösung erwiesen, das einen pH-Wert von etwa 13 aufweist.

Aus praktischen Gründen erfolgt die alkalische Vorbehandlung des Aluminiumoxids zweckmässigerweise durch kontinuierliche Durchströmung der alkalischen Lösung durch das bereits in der Chromatographiesäule gepackte Aluminiumoxid über mehrere Stunden, vorzugsweise etwa 12 bis 16 Stunden, gefolgt von Spülung des so vorbehandelten Aluminiumoxids mit destilliertem Wasser, bis die austretenden Spülungen neutral sind. Um verbleibendes Wasser aus dem vorbehandelten Aluminiumoxid zu entfernen, empfiehlt es sich, die Säule für etwa 4-10 Stunden zwischen 50 und 90°C zu heizen und anschliessend das vorbehandelte Aluminiumoxid noch mit einem niederen Alkan, vorzugsweise n-Heptan, mehrere Stunden, vorzugsweise etwa 8 bis 20 Stunden, zu spülen. Danach kann das so vorbehandelte Aluminiumoxid als stationäre Phase im erfindungsgemässen Verfahren verwendet werden. Die Verwendung des mit Alkali vorbehandelten Aluminiumoxids stellt einen bevorzugten Aspekt des erfindungsgemässen Verfahrens dar.

Damit das als mobile Phase im erfindungsgemässen Verfahrens verwendete Kohlendioxid im überkritischen oder flüssigen Bereich beibehalten wird, müssen gewisse Temperatur- und Druckbedingungen eingehalten werden, und zwar nicht nur bei der Einführung des Kohlendioxids in die mit der stationären Phase gepackte Chromatographiesäule, sondern auch noch während des anschliessenden Eluierens. Das Verfahren wird zweckmässigerweise im Temperaturbereich von etwa 30°C bis etwa 100°C und bei einem Druck von etwa 140 bar bis etwa 320 bar durchgeführt, wobei bei Verwendung des mit Alkali vorbehandelten Aluminiumoxids anstelle des unbehandelten Aluminiumoxids der geeignete Druck bei sonst gleichen Verfahrensbedingungen niedriger ist. Vorzugsweise beträgt der Temperaturbereich etwa 45°C bis etwa 75°C und der diesbezügliche Druckbereich etwa 220 bar bis etwa 260 bar. Die Dichte von Kohlendioxid lässt sich über Druck und Temperatur einstellen, und in dem letzterwähnten Temperatur- und Druckbereich ergibt sich eine optimale Kohlendioxiddichte von etwa 720 kg/m³ bis etwa 850 kg/m³.

Aluminiumoxid bewirkt als stationäre Phase bei der Durchführung des erfindungsgemässen Verfahrens die chromatographische Abtrennung der verschiedenen ungesättigten Fettsäuren oder Fettsäurederivate und, falls vorhanden, gesättigten Fettsäuren oder Fettsäurederivate, und zwar einerseits nach der Kettenlänge (Anzahl der C-Atome) und andererseits nach dem Sättigungsgrad (Anzahl der Doppelbindungen: je höher diese Anzahl, desto niedriger der Sättigungsgrad). Ab einer gewissen Anzahl Doppelbindungen scheint die Selektivität bezüglich des Sättigungsgrades zu überwiegen. Verfügen die abzutrennenden Fettsäuren bzw. Fettsäurederivate über lediglich eine Doppelbindung, wie beispielsweise bei der Erucasäure (22 C-Atome,1 Doppelbindung:"22:1"), so tritt die Selektivität bezüglich der Kettenlänge deutlich in den Hintergrund, und die Fettsäuren bzw. Derivate eluieren vor dem Bereich kürzerer, jedoch höher ungesättigter Fettsäuren bzw. Derivate, wie bei der 20:5-Fettsäure Eicosapentaensäure. Hingegen wird beim Eluieren der 20:5-, 22:5- und 22:6-Fettsäuren (Eicosapentaen-, Docosapentaen- bzw. Docosahexaensäure) die längerkettige Säure gegenüber der kürzerkettigen Säure gleicher Anzahl Doppelbindungen, und die Säure kleineren Sättigungsgrades gegenüber der Säure grösseren Sättigungsgrades, an der stationären Phase jeweils stärker (länger) festgehalten, so dass zuerst die 20:5-Fettsäure, dann die 22:5-Fettsäure, und zuletzt die 22:6-Fettsäure eluiert wird. Gerade im letzteren Bereich (Anzahl C-Atome 20-22 und/oder Anzahl Doppelbindungen 5-6) sind die ungesättigten Fettsäuren grösster Bedeutung auf den Gebieten der Ernährung und der Medizin zu finden, wo es gilt, klare chromatographische Trennungen ohne übermässiges Tailing zu verwirklichen.

Sowohl die Temperatur- und Druckbedingungen, unter denen das erfindungsgemässe Verfahren durchgeführt wird, als auch die Wahl zwischen unbehandeltem Aluminiumoxid und mit Alkali vorbehandeltem Aluminiumoxid als stationärer Phase, üben einen Einfluss auf das Trennergebnis aus. Im allgemeinen zieht eine Temperaturerhöhung oder Druckerniedrigung die Eluate der verschiedenen Fettsäuren zeitlich auseinander, während eine Druckerhöhung oder Temperaturerniedrigung die Eluate zusammenschiebt, so dass das beliebige Variieren dieser Parameter den zeitlichen Ablauf des erfindungsgemässen Verfahrens bestimmen kann. Durch alkalische Vorbehandlung des Aluminiumoxids lassen sich die aktiven Zentren (überwiegend Lewis-Säuren und Basen) auf dessen Oberfläche blockieren, so dass das Aluminiumoxid in seiner Polarität abgeschwächt wird. Die Reihenfolge der Eluate ändert sich nach dieser Behandlung zwar nicht, jedoch verringert sich das Tailing der polaren mehrfach ungesättigten Fettsäuren, und die Elutionszeiten reduzieren sich deutlich. Wird beispielsweise die bei einer Durchführung des erfindungsgemässen Verfahrens bei 65°C und 280 bar auf unbehandeltem Aluminiumoxid zuletzt eluierende 22:6-Fettsäure erst nach etwa einer halben Stunde unter bemerkbarem Tailing eluiert, so erfolgt die Elution bei gleichen Temperatur- und Druckbedingungen auf dem alkalisch behandelten Aluminiumoxid bereits nach nahezu einem Drittel der Zeit und mit stark verringertem Tailing. Aus diesem Grunde wird mit Alkali vorbehandeltes Aluminiumoxid im erfindungsgemässen Verfahren eingesetzt.

Die Detektion der auf der Chromatographiesäule nacheinander eluierenden, in Kohlendioxid gelösten Bestandteile (Eluate) erfolgt sequentiell vorzugsweise über einen UV-Detektor, gefolgt von einem Flammenionisationsdetektor (FID). Der UV-Detektor ermöglicht eine Beurteilung der Bestandteile nach der Anzahl der Doppelbindungen und kann somit zur begrenzten Strukturaufklärung einzelner Bestandteile beitragen. Unter Verwendung des FID wird die Zuteilung der verschiedenen Eluate in die Auffangsgefässe elektronisch besorgt. Derartige Technologie ist an sich bekannt, wie auch die Art und Weise, durch die das Kohlendioxid (durch Dekomprimieren) vom jeweiligen Sammelgut entfernt wird.

Das erfindungsgemässe Verfahren eignet sich ganz bevorzugt zum Gewinnen der 20:5-Fettsäure Eicosapentaensäure sowie der 22:6-Fettsäure Docosahexaensäure, jeweils entweder unmittelbar oder über ein Derivat davon, vorzugsweise den Ethylester.

Die Erfindung wird anhand der nachfolgenden Beispiele veranschaulicht.

### Beispiel 1

### Durchführung des Verfahrens

Für eine Untersuchung des chromatographischen Gewinnens der Bestandteile, insbesondere der ungesättigten Fettsäure-äthylester, aus einem mit Ethanol veresterten Fischöl wird eine Apparatur der Firma Carlo Erba Instruments verwendet (s. Abbildung 1). Die Apparatur wird über eine Kohlendioxid (CO₂)-Flasche kontinuierlich mit mobiler Phase versorgt. Die mobile Phase kann je nach gewählten Druck- und Temperaturbedingungen im überkritischen (bei CO₂ über etwa 31°C und 73 bar) oder auch unterkritischen Bereich betrieben werden.

Dazu wird das CO₂ aus der Flasche in die Kammer der Pumpe gezogen und dort zunächst mittels eines Thermostaten auf -6°C kondensiert. Der Kolben wird über einen Schrittmotor angetrieben und verdichtet so die mobile Phase auf einen gewünschten Druck. Der Druck der Spritzenpumpe sowie die Temperatur des Säulenofens lassen sich per Computer einstellen. Die mobile Phase strömt mit dem gewählten Druck durch die Apparatur und passiert zunächst das Injektionssystem. Hier erfolgt die Aufgabe der Probe über ein Zweiwegeventil in die vorbeiströmende mobile Phase. Die Aufgabezeit lässt sich zwischen 0 und 999 msec beliebig variieren. Das Probenaufgabevolumen beträgt konstant 0,5 µl. Die Probe gelangt dann mit der mobilen Phase auf die Säule. Hier findet aufgrund unterschiedlich starker Wechselwirkungen zwischen der stationären Phase und den einzelnen Bestandteilen der Probelösung eine Auftrennung des Gemisches statt. Die einzelnen Bestandteile des Gemisches werden (im Idealfall) nacheinander von der Säule eluiert. Da sich die Säule in einem Ofen befindet, ist zusätzlich eine Beeinflussung des Elutionsverhaltens über die Temperatur möglich. Die Detektion (Bestimmung) der auf der Säule getrennten Substanzen (u.a. ungesättigten Fettsäure-ethylester) erfolgt sequentiell über einen UV-Detektor und anschliessend mittels eines Flammenionisationsdetektors (FID).

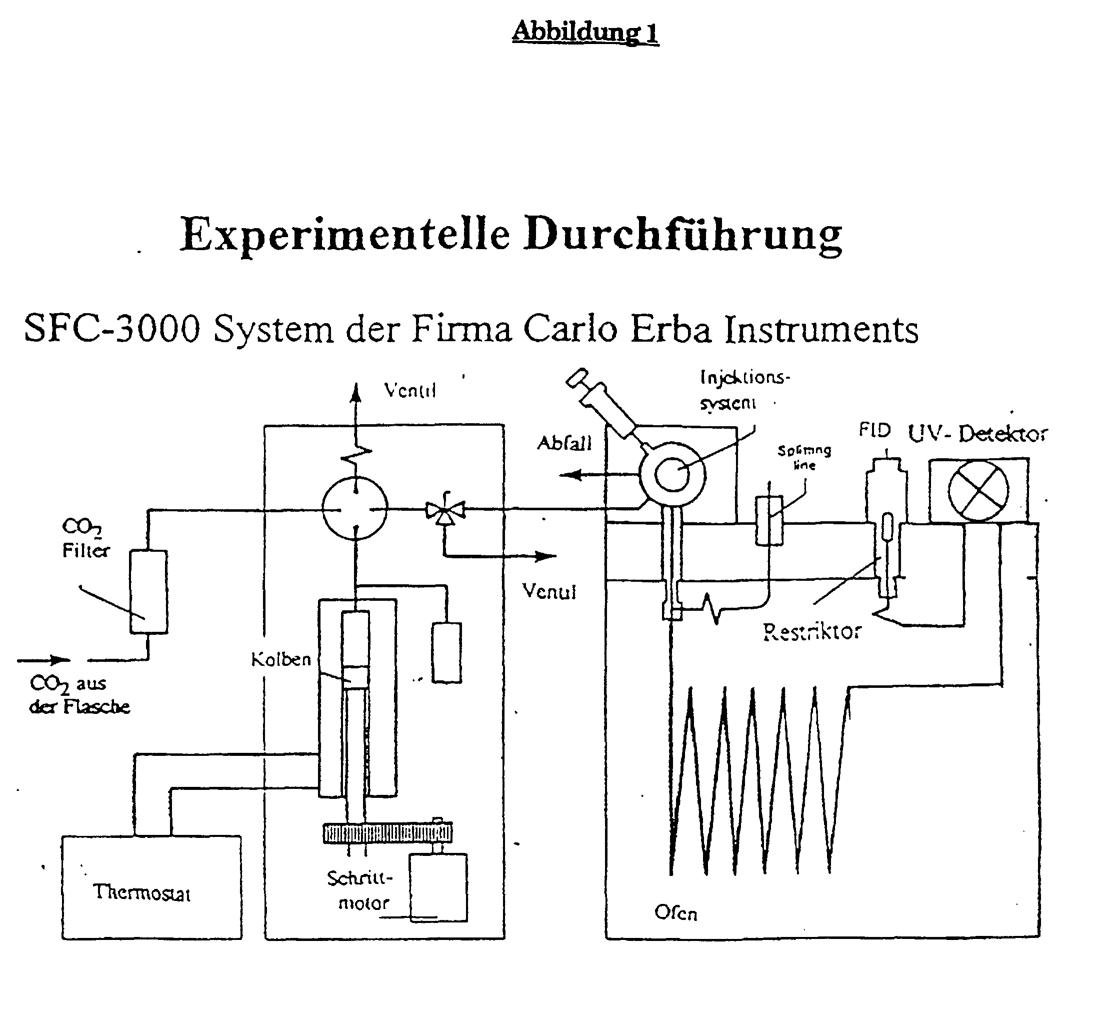

Die nachfolgenden Abbildungen 2 und 3 zeigen die dabei erhaltenen Chromatogramme:

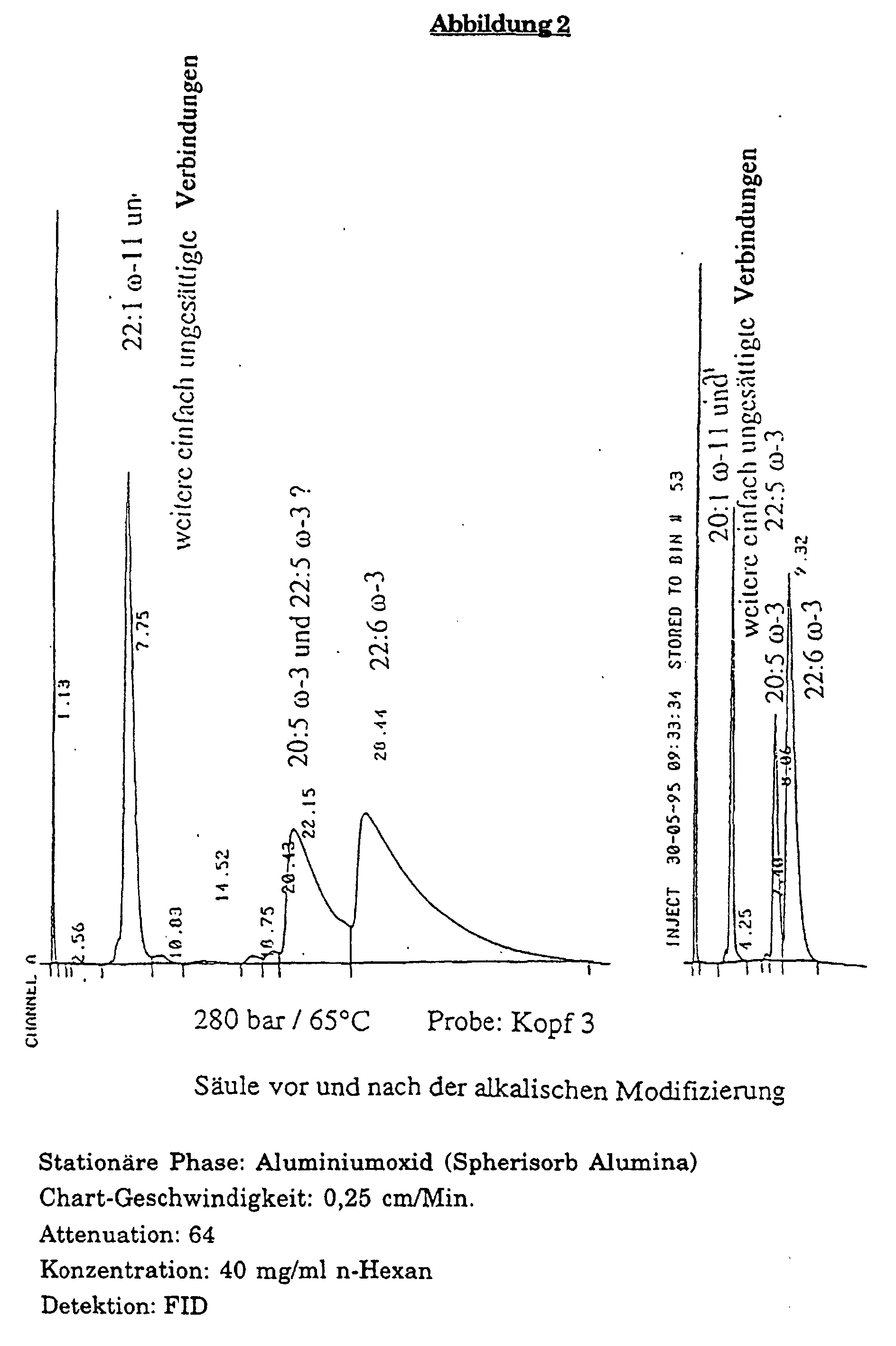

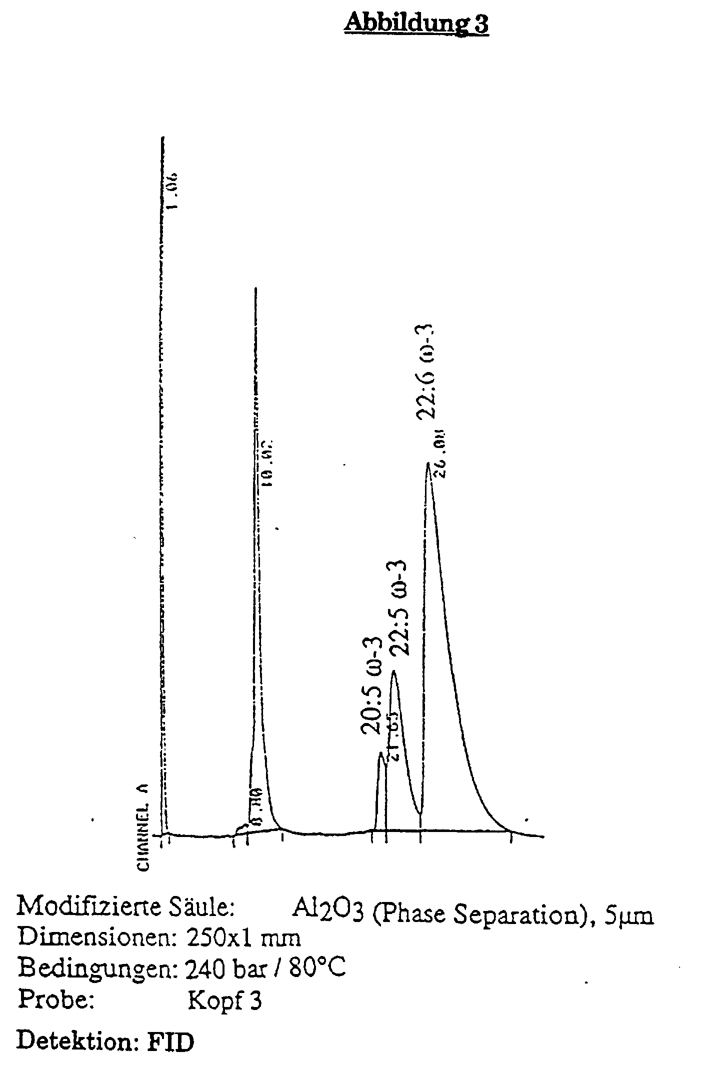

### Stationäre Phase, Chart-Geschwindigkeit, Attenuation sowie Konzentration: wie für Abb. 2.

### Beispiel 2

### Alkalische Vorbehandlung der Aluminiumoxid-stationären Phase

Eine mit Aluminiumoxid gepackte Chromatographie-Säule wird durch mehrstündiges Spülen (12-24 Stunden) mit einem Gemisch aus 0,1 M wässriger Natriumhydroxid-Lösung in Acetonitril (90:10% V/V) behandelt. Der pH-Wert der Lösung beträgt somit 13. Die Säule wird mit destilliertem Wasser neutral gewaschen, für etwa 4-10 Stunden zwischen 50 und 90°C geheizt und anschliessend 12-18 Stunden mit n-Heptan rekonditioniert.

### Beispiel 3

### Durchführung des Verfahrens (weitere Ausführungsform)

Für den Betrieb von Säulen grösseren Innendurchmessers und der damit verbundenen Steigerung des Durchsatzes wird eine Apparatur der Firma New Ways of Analytics (s. Abbildung 4) verwendet. Die Apparatur wird über eine CO₂-Zuleitung kontinuierlich mit mobiler Phase versorgt. Die mobile Phase kann je nach gewählten Druck- und Temperaturbedingungen im überkritischen (bei CO₂ über etwa 31°C und 73 bar) oder auch unterkritischen Bereich betrieben werden. Dazu wird das CO₂, das aus einem CO₂-Behälter in den Thermostaten des Druckmoduls PM-101 strömt, mittels eines Thermostaten auf -6°C kondensiert. Eine pneumatische Kolbenpumpe verdichtet die mobile Phase auf Drücke zwischen 300-400 bar. In einem nachgeschalteten Druckreduktionsmodul PR-102 wird die verdichtete mobile Phase auf den gewünschten Druck reduziert und zusätzlich Druckschwankungen, die aus der Funktionsweise des Druckmoduls PM-101 resultieren, gedämpft. Die mobile Phase strömt mit dem im Druckreduktionsmodul PR-102 gewählten Druck durch die Apparatur und passiert zunächst das Injektionsventil. Hier erfolgt die Aufgabe der Probe über ein Sechswegeventil (Rheodyne 7125) in die vorbeiströmende mobile Phase. Das Probenaufgabevolumen lässt sich durch Wahl entsprechender Probeschleifen von 5 µl bis zu mehreren Millilitern variieren. Die Probe gelangt dann mit der mobilen Phase auf die Säule. Hier findet aufgrund unterschiedlich starker Wechselwirkungen zwischen der stationären Phase und den einzelnen Bestandteilen der Probelösung eine Auftrennung des Gemisches statt. Die einzelnen Bestandteile des Gemisches werden im Idealfall nacheinander eluiert. Da sich die Säule in einem Ofen befindet, ist zusätzlich eine Beeinflussung des Elutionsverhaltens über die Temperatur möglich. Die auf der Säule getrennten Substanzen werden über einen UV-Detektor erfasst und von dort an eine Chromatographiesoftware übermittelt. Die Software ermöglicht das Schalten von Ventilen, die ein Ausschleusen der getrennten Komponenten ermöglichen. Die Entspannung der überkritischen Phase erfolgt abschliessend über die Entspannungseinheit PE-103.

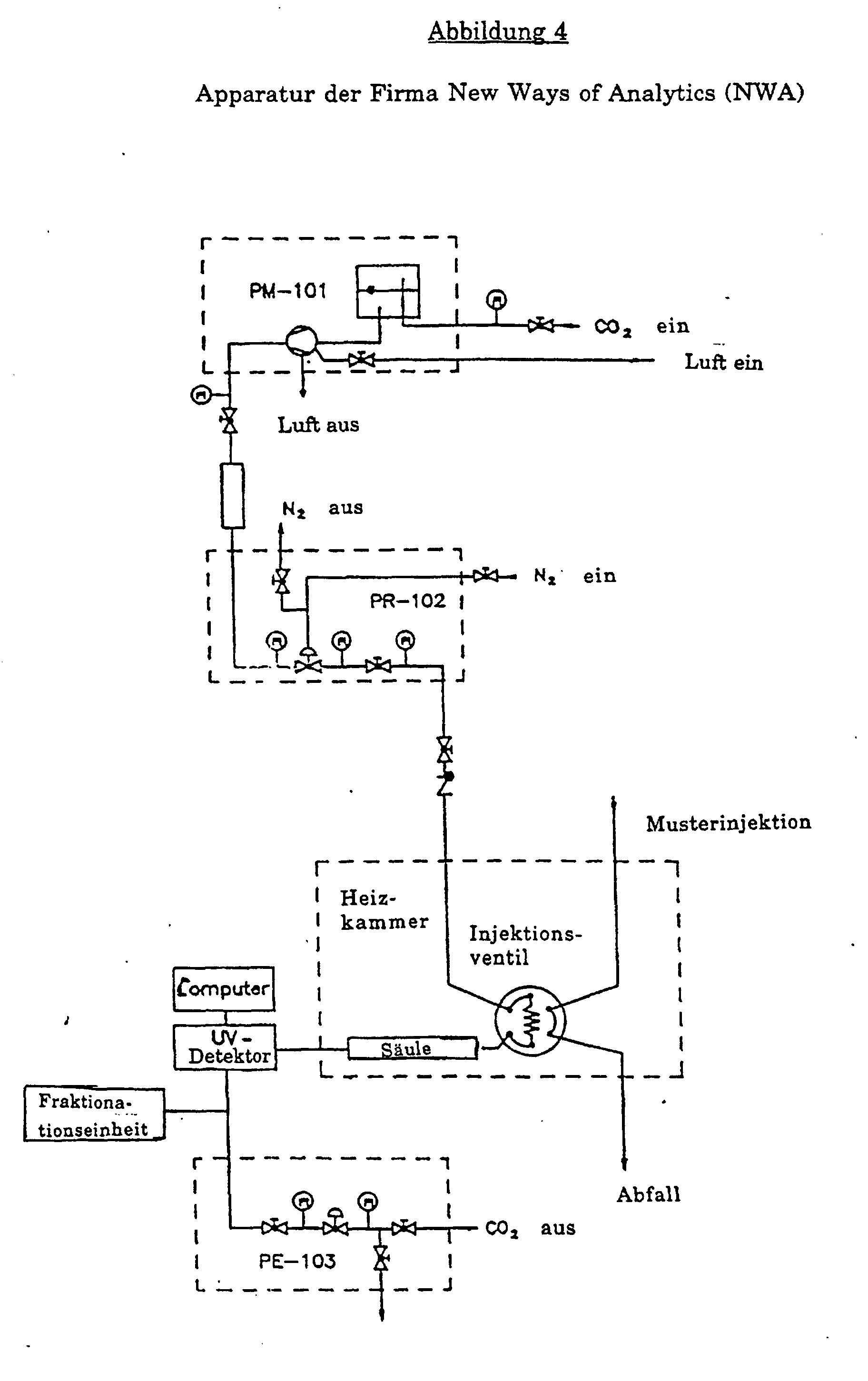

Die nachfolgende Abbildung 5 zeigt das dabei erhaltende UV-Chromatogramm eines gebrochenen Aluminiumoxides, das mittels der NWA-Apparatur gewonnen wurde:

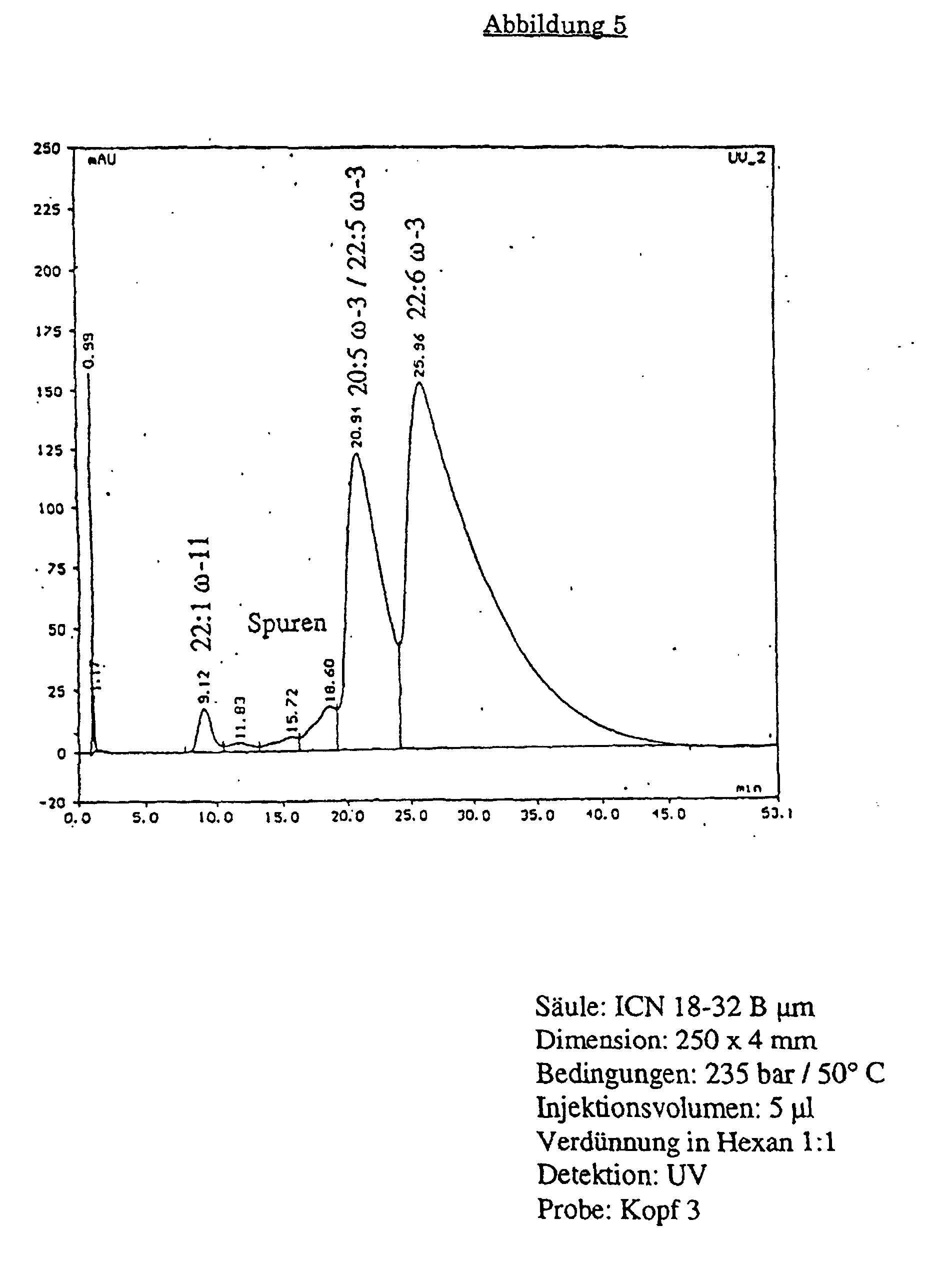

## Patentansprüche

1. Verfahren zum Gewinnen ungesättigter Fettsäuren mit mindestens sechzehn Kohlenstoffatomen im Molekül oder ihrer C₁₋₆-Alkylester aus einem Gemisch von Fettsäuren und/oder Fettsäurederivaten durch Säulenchromatographie mit überkritischem oder flüssigem Kohlendioxid als mobiler Phase, **dadurch gekennzeichnet, dass** man die Fettsäuren in Form ihrer C1-6-Alkylester an mit Alkali vorbehandeltem Aluminiumoxid als stationärer Phase auftrennt und die erhaltenen Ester gewünschtenfalls zu den freien Säuren verseift.

2. Verfahren nach Anspruch 1, **dadurch gekennzeichnet, dass** das mit Alkali vorbehandelte Aluminiumoxid in Form möglichst homogen gepackter, kugelförmiger Partikel vorliegt, deren Partikelgrösse etwa 5 bis 25 µm beträgt.

3. Verfahren nach Anspruch 1 oder 2, **dadurch gekennzeichnet, dass** die Vorbehandlung der stationären Phase durch den Kontakt des partikelförmigen Aluminiumoxids mit einer wässrigen Lösung eines Alkalimetall- oder Erdalkalimetallhydroxids im pH-Bereich von etwa 10 bis etwa 13 über etwa 8 bis etwa 20 Stunden erfolgt.

4. Verfahren nach Anspruch 3, **dadurch gekennzeichnet, dass** zur Einstellung der Viskosität der wässrigen Lösung auf einen arbeitstechnisch praktikablen Wert die Lösung mit einem wassermischbaren oder wasserlöslichen organischen Lösungsmittel, insbesondere mit Acetonitril oder Aceton, ergänzt wird.

5. Verfahren nach Anspruch 4, **dadurch gekennzeichnet, dass** zur Vorbehandlung des Aluminiumoxids ein 10:90 (V/V)-Gemisch von Acetonitril und 0,1 M wässriger Natriumhydroxidlösung verwendet wird.

6. Verfahren nach einem der Ansprüche 1 bis 5, **dadurch gekennzeichnet, dass** man als Gemisch von Fettsäuren und/oder Fettsäurederivaten ein gegebenenfalls im voraus raffiniertes und/oder chemisch behandeltes Fischöl verwendet.

7. Verfahren nach Anspruch 6, **dadurch gekennzeichnet, dass** man als Gemisch von Fettsäuren und/oder Fettsäurederivaten ein im voraus mit einem C₁₋₆-Alkanol verestertes Fischöl, insbesondere ein mit Ethanol verestertes Fischöl, verwendet.

8. Verfahren nach einem der Ansprüche 1 bis 7, **dadurch gekennzeichnet, dass** das Verfahren im Temperaturbereich von etwa 30°C bis etwa 100°C und bei einem Druck von etwa 140 bar bis etwa 320 bar durchgeführt wird.

9. Verfahren nach Anspruch 8, **dadurch gekennzeichnet, dass** der Temperaturbereich etwa 45°C bis etwa 75°C und der diesbezügliche Druckbereich etwa 220 bar bis etwa 260 bar beträgt.

10. Verfahren nach einem der Ansprüche 1 bis 9, **dadurch gekennzeichnet, dass** es zum Gewinnen von Eicosapentaensäure, Docosapentaensäure oder Docosahexaensäure über deren Ethylester verwendet wird.

## Claims

1. A process for the recovery of unsaturated fatty acids with at least sixteen carbon atoms in the molecule or their C₁₋₆-alkyl esters from a mixture of fatty acids and/or fatty acid derivatives by column chromatography with supercritical or liquid carbon dioxide as the mobile phase, **characterized in that** the fatty acids are separated in the form of their C₁₋₆-alkyl esters on aluminium oxide pre-treated with alkali as the stationary phase and, if desired, the esters obtained are saponified to the free acids.

2. A process according to claim 1, **characterized in that** the aluminium oxide pre-treated with alkali is present in the form of bead-like particles packed as homogeneously as possible, the particle size being about 5 to 25 µm.

3. A process according to claim 1 or 2, **characterized in that** the pre-treatment of the stationary phase is effected by contact of the particulate aluminium oxide with an aqueous solution of an alkali metal hydroxide or alkaline earth metal hydroxide in the pH range of about 10 to about 13 over about 8 to about 20 hours.

4. A process according to claim 3, **characterized in that**, in order to adjust the viscosity of the aqueous solution to a value which is practical in use, the solution is supplemented with a water-miscible or water-soluble organic solvent, especially with acetonitrile or acetone.

5. A process according to claim 4, **characterized in that** a 10:90 (v/v) mixture of acetonitrile and 0.1M aqueous sodium hydroxide solution is used for the pre-treatment of the aluminium oxide.

6. A process according to any one of claims 1 to 5, **characterized in that** a fish oil, optionally previously refined and/or chemically treated, is used as the mixture of fatty acids and/or fatty acid derivatives.

7. A process according to claim 6, **characterized in that** a fish oil previously esterified with a C₁₋₆-alkanol, especially a fish oil esterified with ethanol, is used as the mixture of fatty acids and/or fatty acid derivatives.

8. A process according to any one of claims 1 to 7, **characterized in that** the process is carried out in a temperature range of about 30°C to about 100°C and at a pressure of about 140 bar to about 320 bar.

9. A process according to claim 8, **characterized in that** the temperature range is about 45°C to about 75°C and the respective pressure range is about 220 bar to about 260 bar.

10. A process according to any one of claims 1 to 9, **characterized in that** it is used for the recovery of eicosapentaenoic acid, docosapentaenoic acid or docosahexaenoic acid via their ethyl esters.

## Revendications

1. Procédé destiné à obtenir des acides gras insaturés, à au moins seize atomes de carbone dans la molécule, ou leurs C₁₋₆-esters d'alkyle à partir d'un mélange d'acides gras et/ou de dérivés d'acides gras grâce à une chromatographie sur colonne avec du dioxyde de carbone liquide ou supercritique comme phase mobile, **caractérisé en ce qu'**on sépare les acides gras sous forme de C₁₋₆-ester d'alkyle sur de l'oxyde d'aluminium traité au préalable aux alcalis comme phase stationnaire et si souhaité, l'ester obtenu se saponifie en acides libres.

2. Procédé selon la revendication 1, **caractérisé en ce que** l'oxyde d'aluminium traité au préalable aux alcalis se présente sous la forme de particules en forme de billes compactées de façon homogène dont la taille de particule s'élève à environ 5 à 25 µm.

3. Procédé selon la revendication 1 ou 2, **caractérisé en ce que** le traitement préalable de la phase stationnaire a lieu par le contact de l'oxyde d'aluminium sous forme de particules avec une solution aqueuse d'un hydroxyde de métal alcalino-terreux ou de métal alcalin dans l'intervalle de pH d'environ 10 à environ 13 pendant environ 8 à environ 20 heures.

4. Procédé selon la revendication 3, **caractérisé en ce que** pour le réglage de la viscosité de la solution aqueuse sur une valeur techniquement utilisable pour la travailler, la solution est complétée par un solvant organique soluble dans l'eau ou miscible dans l'eau, en particulier avec de l'acétonitrile ou de l'acétone.

5. Procédé selon la revendication 4, **caractérisé en ce que** pour le traitement préalable de l'oxyde d'aluminium, un mélange à 10:90 (V/V) d'acétonitrile et 0,1 M d'une solution aqueuse d'hydroxide de sodium est utilisé.

6. Procédé selon l'une quelconque des revendications 1 à 5, **caractérisé en ce qu'**on utilise une huile de poisson éventuellement préalablement traitée chimiquement et/ou raffinée comme mélange d'acides gras et/ou de dérivés d'acides gras.

7. Procédé selon la revendication 6, **caractérisé en ce qu'**on utilise une huile de poisson préalablement esterisée avec un C₁₋₆-alcanol, en particulier une huile de poisson estérisée avec de l'éthanol, comme mélange d'acides gras et/ou de dérivés d'acides gras.

8. Procédé selon l'une quelconque des revendications 1 à 7, **caractérisé en ce que** le procédé est exécuté dans l'intervalle de température d'environ 30° C à environ 100° C, et sous une pression d'environ 140 bar à environ 320 bar.

9. Procédé selon la revendication 8, **caractérisé en ce que** l'intervalle de température s'élève entre environ 45° C et environ 75° C, et l'intervalle de pression correspondant entre 220 bar et environ 260 bar.

10. Procédé selon l'un quelconque des revendications 1 à 9, **caractérisé en ce qu'**il est utilisé pour obtenir un acide eicosapentaenoique, un acide docosapentaenoïque ou un acide docosahexaenoïque.
